# EUROPEAN PATENT APPLICATION

(11) **EP 1 891 988 A1**
(43) Date of publication of application: **27.02.2008**
(21) Application number: 06118544.3
(22) Date of filing: 07.08.2006
(51) Int. Cl.: A61L 27/28, A61L 27/30, A61L 27/54, A61L 31/08, A61F 2/82, A61C 8/00

(54) **Anisotropic nanoporous coatings for medical implants**

(71) Applicant: DEBIOTECH S.A., 1004 Lausanne (CH)
(72) Inventor: Piveteau, Laurent-Dominique, 1030 Bussigny (CH); Hofmann, Heinrich, 1009 Pully (CH); Neftel, Frédéric, 1005 Lausanne (CH)
(74) Representative: Grosfillier, Philippe

(57) **Abstract**

The present invention relates to porous coatings with controlled structure in the micro and nano-size domain. The coating has a thickness between 10 nanometres and 10 millimetres and its porosity is created in such a way that the pore size distribution is anisotropic.

The invention also concerns processes for fabricating such coatings and objects covered with this coating.

## Description

### Field of invention

The present invention relates to porous coatings with controlled structure in the micro and nano-size domain. In particular, but not exclusively, it relates to coatings with an anisotropic pore size distribution and to processes for fabricating such coatings and to objects obtained covered with this coating.

### State of the art

Failure of a therapy based on systemically administered drugs has many origins, but one classical reason is the inability to achieve the required dose at the site to be treated. This effect is especially true for treatments following implantations. When an implant is placed into the body, it provokes small injuries that will most of the time induce a reaction that is detrimental on the medium to long run. A way to tackle this issue is to use drugs that will locally counteract this reaction.
Drug eluting coatings have created strong interest over the recent years. They are used quite extensively today in cardiology on drug eluting stents for angioplasty and some development is conducted in orthopaedics for hip and knee implants. They can be classified into two major groups. In the first group, the drug to be eluted is mixed to the coating and will be released either in parallel to the dissolution of the coating or by diffusion through the coating. In the second group, the drug is contained into the porosity of the coating that acts as a series of reservoirs. It is released as the body fluid penetrates the porosity and dissolves it. The objective of porous coatings is to reduce the thickness. Thickness of the coating is a critical aspect that has a direct impact on its stability. It is well known from the literature that thick coatings are weaker and have a tendency to break over time. By reducing the thickness of the coating, lifetime is improved, but as a consequence, the amount of drug that can be stored is reduced. By creating small empty reservoirs that can be filled with a drug, this amount can be maintained despite the reduction in thickness.
Prior art shows different ways of creating a porous coating for drug release applications. For example Reed, Looi and Lye (CA 2 503 625) use a differential attack of a metallic alloy. By removing one component of the alloy, they create a porous layer. Brandau and Fischer (US 6,709,379) create the porosity by an electrolytic oxidation combined with an anodization. Herlein, Kovacs and Wolf (EP 1 319 416) create pores at the surface of a metallic stent through electrochemically induced pitting. These holes are then covered with a ceramic layer. In all these cases, the created porosity has the disadvantage of being homogeneous in size, or at least having a homogeneous size distribution. As a result, the loaded amount of drug will be low (small pores) or the release will occur over a short period of time (large pores) i.e. over a few hours.
As a matter of fact, in order to store and release over a few days to a few weeks a large amount of drug, the coating must combine two porosities: one of large size acting as a reservoir and where the drug is stored and another of size similar to the released molecules that acts as a diffusion membrane.

### General description of the invention

Obtaining simultaneously a high drug loading and a slow drug release is achieved with the coating of the present invention which is characterized by the fact that the pores are different in size and disposed in an anisotropic way. Micro-pores are created near the surface of the object and are used to store the drug, while nano-pores are disposed on the outside, towards the free surface of the coating, and act as a release membrane.

As a non-limiting example, we will discuss afterwards only ceramic coatings on a metallic substrate. But the same description can be applied to any type of coating material and the coating can be made of different types of materials: metals, ceramics, polymers, hydrogels or a combination of any of these materials. As the porous coating is in contact with a living body, it is preferably made of a biocompatible material. Depending on the applications this can be, but not exclusively, an oxide, a phosphate, a carbonate, a nitride or a carbonitride. Among the oxide the following ones are preferred: tantalum oxide, aluminum oxide, zirconium oxide or titanium oxide. In some case the coating can also be made of a biodegradable material that will be replaced over time by the living tissue on any type of substrate such as metals, ceramics, polymers or a combination of any of these materials. Metals such as stainless steel, Nitinol, titanium, titanium alloys, or aluminum and ceramics such as zirconia, alumina, or calcium phosphate are of particular interest. It can also be a biodegradable material that will be replace over time by the living tissue. It is therefore understood in that the word ceramic in the following can be replaced by polymer, metal or a combination and the same applies to metal. For differences in the process steps, table 1 describes for most of the possible combinations the modifications that need to be applied.

The coating has a thickness between 10 nanometres and 10 millimetres, preferably between 200 nanometers and 30 micrometers.

The porosity is created in such a way that the size distribution is anisotropic. Preferably, the median value of the pore size distribution in the coating varies from the surface of the object to the free surface of the coating, said free surface being the surface of the coating which is away from the support. Preferably, the mean value of the pore size distribution at the free surface of the coating is less than a few µm.

In another variant, the coating is made of distinct sub-layers with distinct porosity size distributions. In this case, one of the sub-layers has a mean pore size distribution of less than a few µm and preferably, the sub-layer with the smallest mean pore size distribution is located near the free surface of the coating. Preferably, the two porosity mean pore diameters differ by a factor 5 to 10.

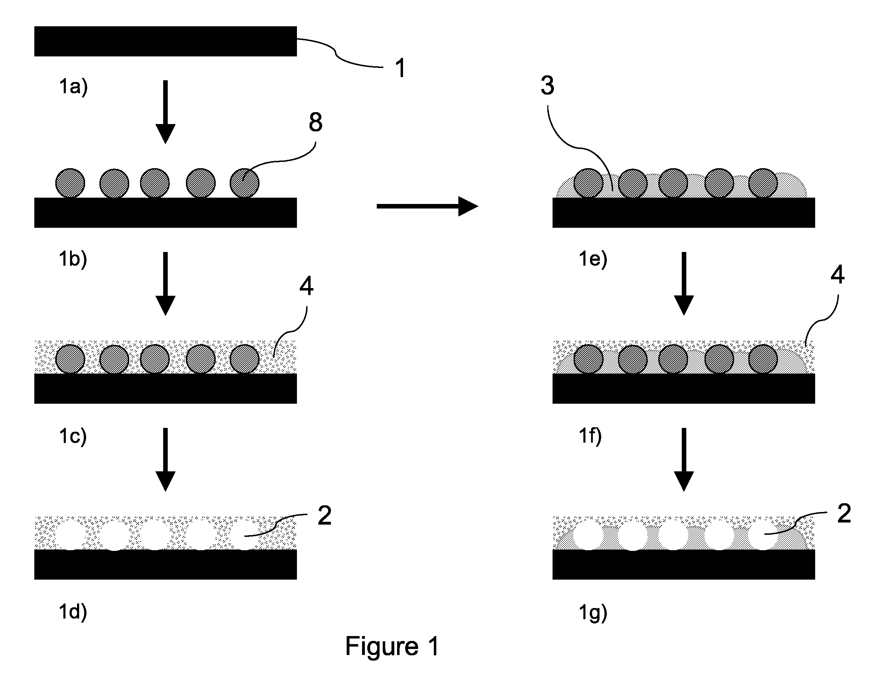

In one possible embodiment micrometer size cavities are created by depositing a template onto the implant. This template is made, for example, of mono disperse polystyrene particles that are deposited by dip coating onto the substrate. It is then partially covered with the ceramic while the diffusion membrane is produced by adding a second layer of nano-porous ceramic. Finally the template materials are removed by a thermal treatment and cavities are created. This embodiment is schematically shown in cross section in figure 1. A ceramic film made of two sublayers is coated onto a metallic substrate (1). The lower layer is made of micropores (2) (pores with diameter in the micron range) embedded in a dense ceramic (3). The upper layer is made of a nano-porous ceramic (4) (ceramic with pores in the nano-meter range).

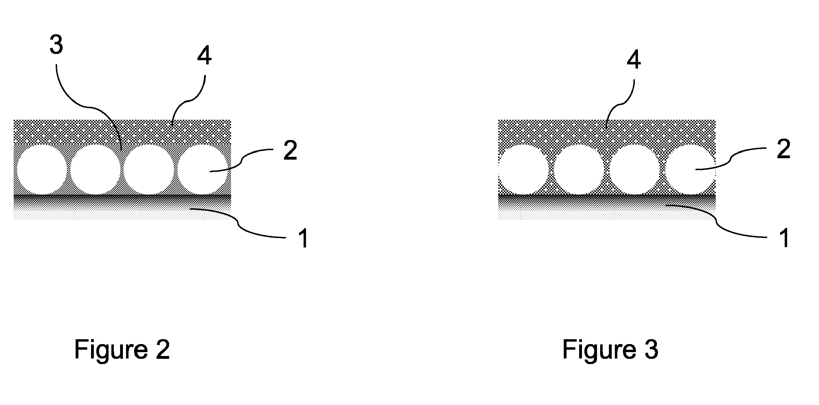

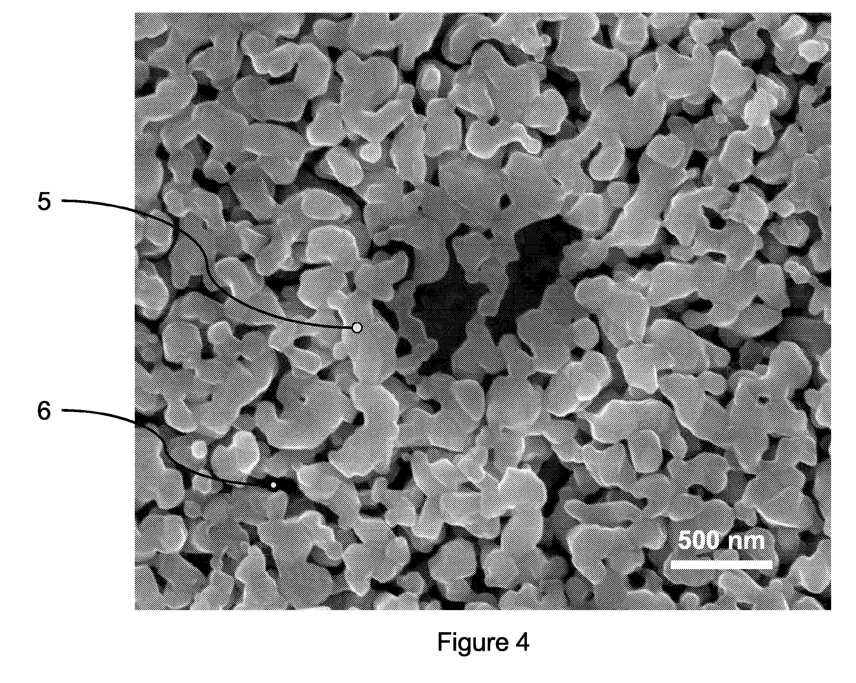

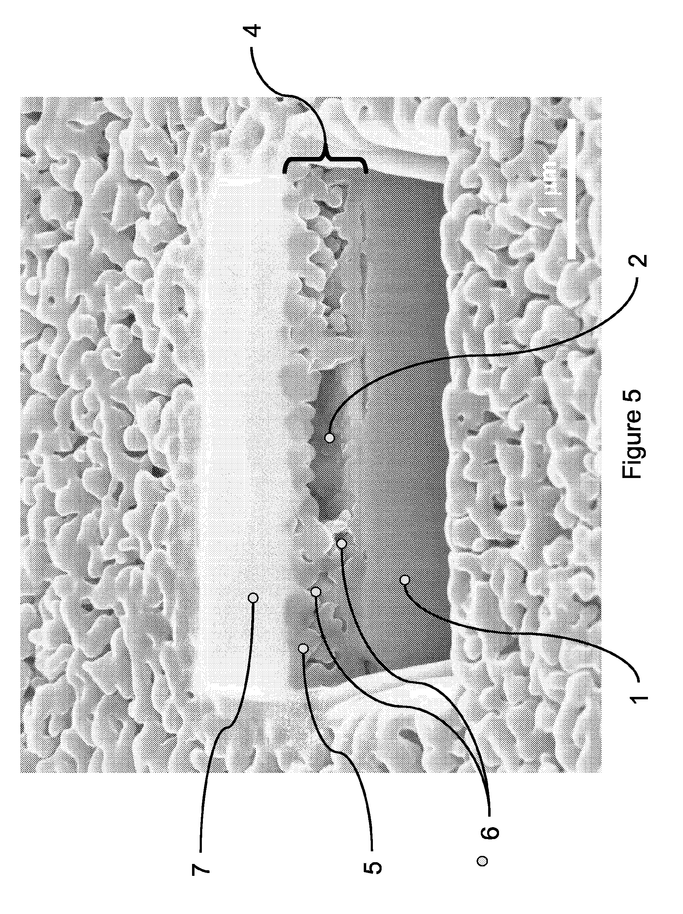

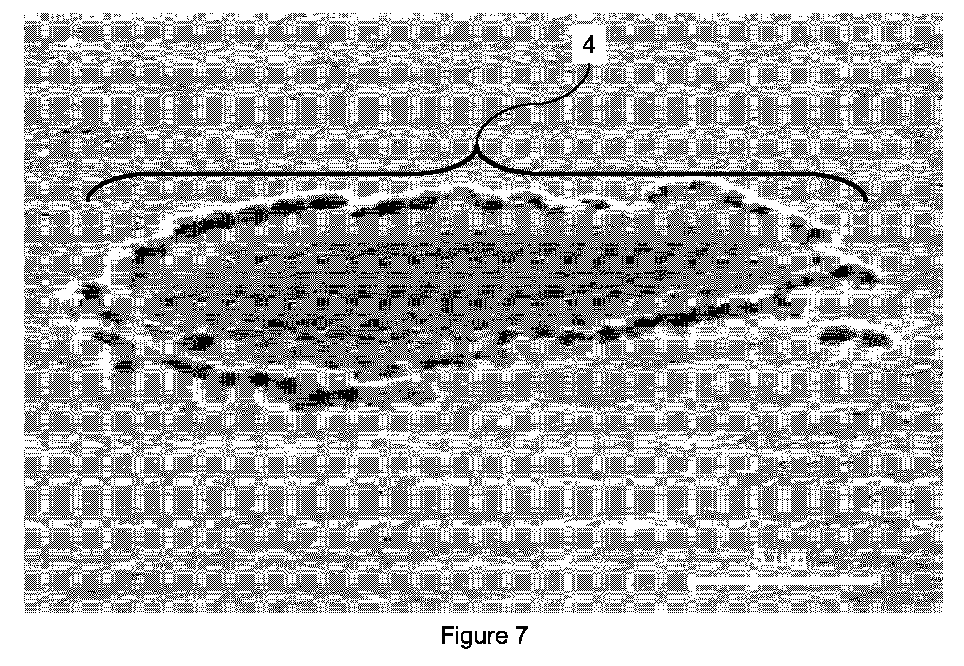

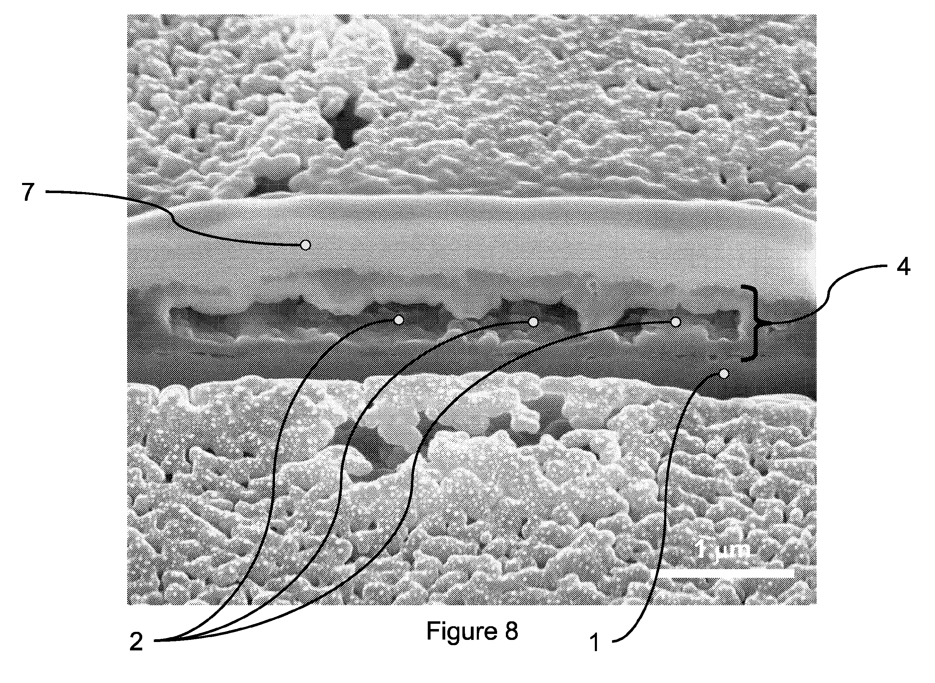

The figure 2 and 3 shows a schematic view of the cross section of an other possible embodiment of the invention. In that embodiment the micro-pores (2) are embedded into the nano-porous ceramic (4). A top view photograph of this second embodiment is shown in figure 4. The micro-pore (5) with a diameter of one micrometer can be seen through the top nano-porous layer (6). The difference in contrast (black shape of the micro-pore) is due to a loading effect of the poorly conductive ceramic. The same coating is shown in figure 5 as a cross section photograph. Under a platinum bar 7 (used for sectioning the coating) the different layers of the coating can be distinguished. On the substrate (1), one has an empty pore (2) created by an eliminated temporary particles, said pore being surrounded by a porous structure (4) made of nano-particles (5) and nano-pores (6). The porosity is created in a controlled way with the larger pores located next to the substrate and the smaller pores towards the free surface of the coating. This controlled distribution creates an anisotropic porous structure in the coating. If several of these particles are placed together, one gets connected micro-cavities. Figure 7 shows a top view photograph and figure 8 a cross section photograph of such a structure.

In a further variant, the pores are made hydrophobic in order to be filled by a lipophilic solution which can be slowly released in an aqueous medium or wherein the pores are made hydrophilic in order to be filled by a hydrophilic solution.

### General coating process

The following is a description of two possible processes to produce these anisotropic coating. The steps for these processes are shown in figure 1.
A first embodiment of the coating process comprises the following steps:
1) a support or substrate having a surface is provided (figure 1a));
2) at least one mono-layer of temporary particles is deposited onto the support or substrate to form a temporary particles construction or template (figure 1b));
3) a coating is deposited onto the support or substrate wherein the coating is nano-porous (figure 1c));
6) the temporary particles are eliminated thus forming pores in order to obtain a structure with a porosity having an anisotropic pore size distribution (figure 1d));
7) the coating is consolidated through a fixation step.

In a second embodiment of the process, the coating is made of two different coatings, a first dense coating not entirely covering said temporary particles (figure 1e)), and by a second nano-porous coating (figure 1f)), said first coating being dried before deposition of said second porous coating. Finally particles are eliminated forming pores with an anisotropic size distribution (figure 1g)) and the coating is consolidated through a fixation step. In this embodiment, the first coating forms a dense structure around the temporary particles.

In the present text, the term "eliminating" is used in a broad sense. It covers any commonly used terms related to an important change in the particle morphology, such as for example disintegration, dissolution or removal. For instance, but not exclusively, elimination of the temporary particles may comprise a thermal step, a chemical step, a mechanical step, an electro-mechanical or an irradiation step. In the case of a thermal, a chemical or an irradiation step, the temporary particles are either completely destroyed or only partially, e.g. the particles can be made hollow. In the case of a mechanical step, the temporary particles can be mechanically removed. In the case of an electro-mechanical step (e.g. sonication or ultrasonic vibrations), the particles can be swelling (e.g. by use of polymeric particles, such as PLGA) or disintegrated.

The term "temporary" has to be understood as "present only for a limited time during the process". Temporary particles can be viewed as templates that create the tridimensional structure and porosity of the coating.

The expression "mono-layer of particles" means that the particles are at the same level relatively to the surface of the support. For each mono-layer, no particle will sit on top of another.

### Temporary particles deposition or templates

Preferably, in the present invention, this step is carried out by dip-coating. The temporary particles are in a solution (for example water) and the substrate is dipped in said solution. As is known in the art, the density of particles present on the substrate will depend on the concentration of particles in the solution, the rate of withdrawal of the substrate and also the surface treatment of the substrate. All these parameters may be adjusted by the skilled man to attain the desired density of particles on the substrate.

The temporary particles and the coating may be deposited together as a slurry.

### Particles

The diameter and the shape of the temporary particles can be chosen arbitrarily. But a preference is given for homogeneous particles in shape and size. The chemical composition of the particles is also free, but it is preferably selected in the group of polymers, starch, silica, metals or biological materials such as cells. A preference is given for polymers materials with a spherical shape and homogeneous diameter: mono-disperse polymer beads. For example, polystyrene bead may be advantageously used. They are readily available in numerous sizes and are very consistent in size. Alternatively, biocompatible polymers (e.g. PLGA or Poly Lactide Glycolide Acid type) can also be used.

When deposited on the support temporary particles can either be in contact with each other or separated by some empty space. When in contact, the contact surface size can be modified by changing the surface chemistry and surface affinity of the particles. It can be increase by using wettable particles or reduced to a point-like contact when using non-wettable particles such as Teflon.

Using hydrophilic and / or hydrophobic temporary particles allows the creation of various structures in the coating. Before the deposition of the temporary particles, the substrate is locally covered with a hydrophilic respectively a hydrophobic layer. In this way specific zones are adapted to fix temporary particles with a similar surface affinity while attachment on the other zones is prevented. In the case of a stent, it may be advantageous to only coat regions which are less subject to deformations; alternatively it may be advantageous to only coat regions which are in contact with the intima of the vessel to target the release of drug to prevent proliferation or inflammation. In the case of bone or dental implants, it may be advantageous to select regions where bone ingrowth should be favored and where it should be hindered.

### Coating deposition

Different procedures can be considered for the coating deposition. They are chosen according to the coating precursors that are used as well as to the desired properties of the coating. A few examples are given below:

A first procedure to deposit the coating onto the substrate uses a mixture of nanoparticles in a solvent such as for example water as coating precursor. The substrate is dipped into the precursor mixture and pulled out at a controlled speed. The thickness of the coating varies with the viscosity of the mixture and with the pulling speed.
Another procedure uses a sol obtained through hydroxylation and partial condensation of a metallic alkoxyde as coating precursor. Again, the precursor can be coated onto the substrate using either dip or spin coating.

In an other procedure, a slurry containing both the removable particles and the coating precursor dissolved in, for example, water is coated onto the substrate.

In all cases the coating can be deposited in several steps or sublayers. Between the depositions of each sub-layer the solvent of the coating precursor can be partially or fully removed by, for example, a thermal treatment. This approach permits the formation of thicker, crack-free coatings. The composition of the coating precursor can also be modified between each step. This allows the creation of coatings with variable chemical composition. For example, the chemical composition of the coating can be very similar to that of the substrate at the coating / substrate interface and can be very compatible at the interface with the body.

Using nanopowders or a sol-gel approach for producing coatings offers the advantage of reducing the necessary temperature for obtaining crystalline coatings. This is particularly favorable for metallic substrates that may go through phase transitions when thermally treated and therefore lose part of their mechanical or shape memory properties.

According to the first embodiment of the process, there is only the deposition of one single first coating entirely covering the temporary particles, said single coating having a nano-porous structure. Such porous structure thus allows the elimination of the particles as indicated here under.

According to the second embodiment of the process, the coating comprises in fact two coatings, a first coating which is treated after deposition (for example dried), said first coating not covering entirely the deposited particles, and then a second coating having a porous structure as in the first embodiment of the process.

Typically, these coatings according to the two embodiments indicated above are realized using nano-powders or by a sol-gel route, known per se in the art. Of course, other methods for creating a porous structure may be envisaged in the present invention.

### Particles removal

The elimination of the temporary particles can be achieved by different methods such as for example, but not exclusively, a thermal, a chemical, a mechanical, an electro-mechanical, a photo-chemical or an irradiation step. It can also take place at different stages of the process, before and / or during and / or after the fixation step, depending on the coating requirements

### Fixation

Any appropriate method can be used for the fixation step. Advantageously a drying step is used.

The coating fixation step can take place before the particle elimination step or it can take place simultaneously with the particle elimination step or even after the particle elimination step.

For ceramics this can be sintering where the crystalline phase is formed. For a polymer this can be a photo-chemically (by visible of UV light), a thermally or chemically induced polymerisation. For metals or for certain ceramics this can be a thermal treatment under controlled (neutral or reducing) atmosphere.

### Object

The processes previously discussed allow the manufacturing of coatings and objects carrying such coatings with specific and original features.

### Application

A major application for these objects, as can be readily understood from the different embodiments and variants described above, is in the field of medical implants. Of particular interest are stents, orthopedic and dental implants. The porosity can be used as a drug reservoir that will release its content in a controlled way over time or it can be used to favor tissue ingrowth and therefore increase the mechanical interlocking between the implant and the living tissue.

For stents the coating can be loaded with one or several drugs. It can be a combination of the following drugs given as non-exclusive examples: an anti-proliferative agent, an anti-coagulation substance, an anti-infectious, a bacteriostatic substance.

The object can also be an orthopedic or dental implant wherein the pores may be adapted in the same manner as for the stent discussed above. In such case, the porosity obtained can either be of interest to store growth factors such as bone growth factors, increase biocompatibility or create regions where bone or cartilaginous tissue can grow and attach in a solid manner to the implant. This can also be achieved by filling the cavities with resorbable bioactive ceramics such as calcium phosphates.

Accordingly the support can be made of metal, of ceramic or polymer. It can also be made of a biodegradable material.

In this application, the coating may comprise non-porous domains. Such domains may have a minimal dimension larger than 10 micrometres and a maximal dimension smaller than 10 millimetres. In a variant, these domains have a minimal dimension larger than 100 micrometers. In another variant, these domains have a maximal dimension smaller than 1 millimetre.

The pore size may also be adapted for diffusing beads, particles or polymers containing an active substance which can be slowly released.

Alternatively the beads or particles can emit an irradiation. Advantageously, in such case, the beads or particles shall remain within the cavities.

### Short description of the figures

Figure 1 shows the different process step for two possible embodiments.
Figure 2 is a possible embodiment of the coating.
Figure 3 is an other possible embodiment of the coating.
Figure 4 is a first photograph of an object which has undergone the process according to the invention.
Figure 5 shows a second photograph of an object which has undergone the process according to the invention.
Figure 6 is a third photograph of an object which has undergone the process according to the invention.
Figure 7 is a fourth photograph of an object which has undergone the process according to the invention.
Figure 8 is a fifth photograph of an object which has undergone the process according to the invention.

Table 1 summarizes different possibilities for manufacturing a porous surface according to the present invention.

### Figures and tables

The present invention will be more fully understood from the following figures and table:

**Table 1**

| **Template's materials** | **Coating's** | **Consolidation material techniques** | **Elimination methods of the template: before, during or after the consolidation step** |
|---|---|---|---|
| Polymer | Polymer | UV -, Thermal - Polymerisation | *After -* Chemical selective dissolution |
| polymer | Metal | Thermal - Annealing, ... | *Before or after* - Chemical selective dissolution |
| | | | *Before or after -UV*-irradiation, oxygen plasma |
| | | | *During* - Pyrolsis *After* - Mechanical (ultrasonic,...) |
| polymer | Ceramics | Thermal - Sintering | *Before* - Chemical selective dissolution |
| | | | *Before* - UV-irradiation, oxygen plasma |
| | | | *During* - Pyrolysis |
| Metal | polymer | UV-, Thermal - Polymerisation | *After* - Chemical selective dissolution |
| Metal | Ceramics | Thermal - Sintering | *Before* - Chemical selective dissolution |
| Ceramics | Polymer | UV-, Thermal - polymerisation Polymerisation | *After* - Chemical selective dissolution |
| | | | *After-* Mechanical (ultrasonic,...) |
| Ceramics | Metal | Thermal - Annealing, ... | *Before or after* - chemical selective dissolution |
| | | | *After-* Mechanical (ultrasonic,...) |
| Ceramics | Ceramics | Thermal-Sintering | *Before or after* - chemical selective dissolution |

In figure 1, the different steps of the coating process are represented. 1a) shows the substrate (1) before the process. The first step 1b) is the deposition of temporary particle (8) or template. As indicated in the description and in table 1, the particles can be made of several materials and can be deposited in a dispersed layer or in a dense layer. Figures 1c) and 1d) show one possible coating process corresponding to the embodiment shown in figure 3, while figure 1e) to 1g) show an other coating process that corresponds to the embodiment shown in figure 2. In the first approach, a nano-porous coating (4) is deposited around and above the template particle 1b), while in the other approach, a dense layer (3) is first deposited around the template particles 1e) without covering them and an nano-porous layer (4) is deposited above this dense layer and the template particles 1f). In both cases 1d) and 1g), the template particles are finally removed to create the micro-pores (2) used to load the drug. The coating thus forms a reservoir structure able to contain a drug for example, which can be released little by little through the porous layer.

Figure 2 is a schematic drawing of the cross section of a possible embodiment of the present invention. A ceramic film made of two sublayers is coated onto a metallic substrate (1). The lower layer is made of micro-pores (2) (pores with diameter in the micron range) embedded in a dense ceramic (3). The upper layer is made of a nano-porous ceramic (4) (ceramic with pores in the nano-meter range).

Figure 3 is an other embodiment of the invention where the micro-pores (2) are embedded into the nano-porous ceramic (4), without the dense ceramic layer (3) shown in the embodiment of figure 2.

Figure 4 is picture of a top view of a structure made according to the first embodiment of the process of the present invention. One sees a micro-pore (black circular shape) with a diameter of one micrometer covered by the nano-porous layer (5) and (6).

Figure 5 shows a cross section of the coating, underneath a platinum bar (7) (used for sectioning the coating) showing the different layers of the coating. On the substrate (1), one sees an empty micro-pore (2) left by an eliminated temporary particles, said pore being surrounded by a nano-porous structure (4) made of nano-particles (5) and nano-pores (6).

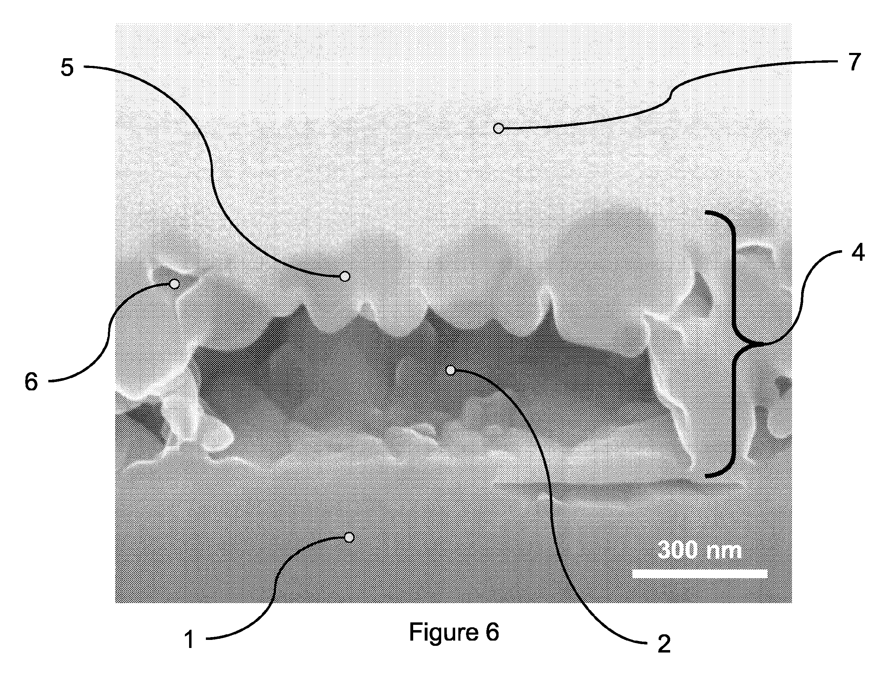

Figure 6 is a closer view of the micro-pore (2), showing the substrate (1) and the nano-porous structure (4) with the nano-particles (5), the nano-pores (6) and the platinum bar (7).

Figure 7 is a top view of the coating where a group of closely packed particles have created a series of interconnected empty micro-pores covered by a nano-porous structure (4).

Figure 8 is a cross section of the coating shown in figure 7. On the substrate (1), one sees a series of empty interconnected micro-pores (2) surrounded by a nano-porous structure (4) and covered by the platinum bar(7).

## Claims

1. A coating deposited on an object with a surface having a thickness between 10 nanometres and 10 millimetres, wherein this coating is porous with an anisotropic pore size distribution.

2. A coating as defined in **claim 1,** wherein the median value of the pore size distribution in the coating varies from the surface of the object to the free surface of the coating.

3. A coating as defined in **claim 2,** wherein the median value of the pore size distribution in the coating decreases from the surface of the object to the free surface of the coating.

4. A coating as defined in **claim 1,** wherein the mean value of the pore size distribution at the free surface of the coating is less than 1 µm.

5. A coating as defined in **claim 1 to 3,** wherein the coating is made of distinct sub-layers with distinct porosity size distributions.

6. A coating as defined in **claim 5,** wherein one of the sub-layers has a mean pore size distribution of less than 1 µm.

7. A coating as defined in **claim 6,** wherein the sub-layer with the smallest mean pore size distribution is located near the free surface of the coating.

8. A coating as defined in **claim 5,** wherein the two porosity mean pore diameters differ by a factor 5 to 10.

9. A coating as defined in **claim 5,** wherein the two porosity mean pore diameters differ by a factor of 100 or more.

10. A coating according to any one of the preceding claims, wherein the pore sizes are adapted for storage and diffusion of an active substance for medical purposes.

11. A coating according to **claim 10,** wherein the substance is a drug, an anti-coagulation substance, an anti-proliferative substance, an antibiotic substance, a bacteriostatic substance or a growth factor.

12. A coating according to **any one of the preceding claims**, wherein the pores are adapted to receive cells.

13. A coating according to **any one of the preceding claims**, wherein the coating thickness is at least equal to 200 nanometres.

14. A coating according to **any one of the preceding claims,** wherein the coating thickness is less than 30 micrometres.

15. A coating according to **any one of the preceding claims**, wherein the coating is made of a ceramic such as an oxide, a phosphate, a carbonate, a nitride or a carbonitride, or a metal, or a polymer, or an hydrogel.

16. A coating according to **claim 15**, wherein the oxide is titanium oxide, tantalum oxide, silicon oxide, iridium oxide or zirconium oxide.

17. A coating according to **any one of the preceding claims,** wherein said coating is obtained from a nanopowder, or from a liquid precursor such as a solution or a sol.

18. A coating according to a**ny one of the preceding claims**, wherein the pores are made hydrophobic in order to be filled by a lipophilic solution which can be slowly released in an aqueous medium or wherein the pores are made hydrophilic in order to be filled by a hydrophilic solution.

19. A coating according to **any one of the preceding claims,** wherein the coating is made of a biodegradable material.

20. An object with a coating as defined in **any one of the preceding claims.**

21. An object according to **claim 20,** wherein this object is a medical implant.

22. An object according to **claim 21,** wherein this object is a stent.

23. An object according to **claim 21,** wherein this object is an orthopaedic implant.

24. An object according to **claim 20,** wherein the support is made of metal.

25. An object according to **claim 20,** wherein the support is made of ceramic.

26. An object according to **claim 20,** wherein the support is made of polymer.

27. An object according to **claim 20,** wherein the support is made of a biodegradable material.

28. An object according to **anyone of the preceding claims,** wherein the coating comprises non-porous domains

29. An object according to **claim 28,** wherein these domains have a minimal dimension larger than 10 micrometres and a maximal dimension smaller than 10 millimetres.

30. An object according to **claim 29,** wherein these domains have a minimal dimension larger than 100 micrometers.

31. An object according to **claim 29,** wherein these domains have a maximal dimension smaller than 1 millimetre.

32. A process for manufacturing an anisotropic porous coating with a pore size distribution in the micro or nano-size domain on a support of an object according to one of the preceding claims, **characterized by** the following steps:
- providing a support having a surface,
- depositing on said surface at least one first mono-layer of temporary particles,
- depositing at least a coating on said temporary particles wherein said coating is porous,
- eliminating said temporary particles forming pores, to obtain a structure with a porosity with an anisotropic pore size distribution, said process furthermore comprising a coating fixation step.

33. Process according to **claim 32,** wherein said coating is made by a first coating not covering entirely said temporary particles, and by a second porous coating, said first coating being dried before deposition of said second porous coating.

34. Process according to **claim 33,** wherein the first coating forms a dense structure around the temporary particles.

35. Process according to **claims 32 to 34,** wherein said temporary particles have at least two different diameters.

36. Process according to **any one of claims 32 to 35,** wherein said temporary particles are deposited on the support in such a way as to be in contact between each other.

37. Process according to **any one of claims 32 to 36,** wherein the temporary particles and the coating are deposited together as a slurry.

38. Process according to **any one of claims 32 to 37,** wherein said temporary particles materials are selected in the group of polymers, starch, ceramics, silica, metals or biological material.

39. Process according **to claim 38,** wherein the polymer particles are polystyrene beads.

40. Process according to **any one of claims 32 to 39,** wherein the substrate is first partially or fully covered by a hydrophobic respectively hydrophilic layer creating hydrophobic respectively hydrophilic domains on the substrate.

41. Process according to **any one of claims 32 to 40,** wherein hydrophobic respectively hydrophilic particles are used to build the mono-layer of temporary particles exclusively onto the hydrophobic respectively hydrophilic domains of the substrate.

42. Process according to **any one of claims 32 to 41,** wherein the coating fixation step takes place before the particle elimination step.

43. Process according to **any one of claims 32 to 41** wherein the coating fixation step takes place simultaneously with the particle elimination step.

44. Process according to **any one of claims 32 to 41,** wherein the coating fixation step takes place after the particle elimination step.

45. Process according to **any one of claims 32 to 44**, wherein said temporary particles are eliminated from the layer by a thermal step, a chemical step, an electro-chemical step, a photo-chemical, a mechanical or irradiation step.

46. Process according to **any one of claims 32 to 45,** wherein said fixation step comprises a drying step.

47. Process according to **any one of claims 32 to 46**, wherein the fixation step is a temperature, UV, chemical, photo-chemical or a polycondensation step.

48. Process according to **claim 47**, wherein fixation step is followed by an anodisation step.

49. Process according to **any one of claims 32 to 48,** wherein the pores are then filled by a dip-coating step.

50. Process according to **any one of claims 32 to 49**, wherein the pores are made hydrophobic in order to be filled by a lipophilic solution.

51. Process according to **any one of claims 32 to 49,** wherein the pores are made hydrophilic in order to be filled by a hydrophilic solution.
